# EUROPEAN PATENT APPLICATION

(11) **EP 1 396 809 A1**
(43) Date of publication of application: **10.03.2004**
(21) Application number: 03006243.4
(22) Date of filing: 20.03.2003
(51) Int. Cl.: G06F 19/00, A61B 5/00

(54) **Telediagnosis system**

(30) Priority: 09.08.2002 JP 2002232616
(71) Applicant: Colin Corporation, Komaki-shi, Aichi-ken (JP)
(72) Inventor: Nunome, Tomohiro, Komaki-shi, Aichi-ken (JP)
(74) Representative: Winter, Brandl, Fürniss, Hübner, Röss, Kaiser, Polte Partnerschaft

(57) **Abstract**

A telediagnosis system (10) for a doctor to make a diagnosis on a patient remote from the doctor, the system comprising an image taking device (14b, 18e, 28, 18i, 20) which takes an image of the patient; an input device (14e) which is operable for inputting a set of patient identifying information that identifies said patient; a memory device (18f) which stores respective images of a plurality of patients, and a plurality of sets of patient identifying information that identify the plurality of patients, respectively; a patient identifying means (22) for identifying the patient by checking the set of patient identifying information inputted through the input device, against the plurality of sets of patient identifying information stored by the memory device; an image selecting means (24) for selecting, from the respective images of the plurality of patients stored by the memory device, the image of the patient identified by the patient identifying means; and a doctor-side terminal device (18) including a display device (18a, 26) which displays the image of the patient selected by the image selecting means, and the image of the patient taken by the image taking device, such that the two images are comparable with each other by the doctor.

## Description

### BACKGROUND OF THE INVENTION

### Field of the Invention

The present invention relates to a telediagnosis system that enables a doctor remote from a patient to make a diagnosis on the patient.

### Related Art Statement

In a telediagnosis system for a doctor remote from a patient to make a diagnosis on the patient, a patient-side terminal device that is disposed in, e.g., the patient's house and a doctor-side terminal device that is operated by the doctor are connected to each other by a communication line, so that physical information, such as blood pressure, that is obtained from the patient is transmitted to the doctor and a diagnosis made by the doctor based on the physical information is transmitted to the patient.

Since the doctor makes the diagnosis based on not just the physical information sent from the patient's terminal device but also the patient's past clinical chart, the doctor needs to identify the patient. To this end, such a method has been generally used which automatically identifies the patient's terminal device connected to the doctor's terminal device, because it is easy and reliable.

However, to automatically identify the patient's terminal device connected to the doctor's terminal device does not mean to identify the patient, but it assumes that the patient's terminal device identified corresponds, one by one, to the patient. For example, in the case where a patient's terminal device is disposed in the patient's house, it can be assumed that the patient's terminal device is used by the patient, and accordingly it can be said that to identify the patient's terminal device means to identify the patient. However, in the case where a single patient's terminal device can be used by a plurality of patients who live in, e.g., an old-age home, it cannot be said that to identify the patient's terminal device connected to the doctor's terminal device means to identify the patient. Thus, the above-indicated patient's terminal device cannot be used in a facility in which a plurality of patients live.

In addition, there has also been used a method in which a patient is identified by inputting, in a patient's terminal device, an identification number given to the patient. However, an incorrect identification number may be inputted in the patient's terminal device. Thus, this method is not sufficiently reliable, either.

### SUMMARY OF THE INVENTION

It is therefore an object of the present invention to provide a telediagnosis system that can correctly identify a patient.

The above object has been achieved by the present invention. According to a first aspect of the present invention, there is provided a telediagnosis system for a doctor to make a diagnosis on a patient remote from the doctor, the system comprising an image taking device which takes an image of the patient; an input device which is operable for inputting a set of patient identifying information that identifies the patient; a memory device which stores respective images of a plurality of patients, and a plurality of sets of patient identifying information that identify the plurality of patients, respectively; a patient identifying means for identifying the patient by checking the set of patient identifying information inputted through the input device, against the plurality of sets of patient identifying information stored by the memory device; an image selecting means for selecting, from the respective images of the plurality of patients stored by the memory device, the image of the patient identified by the patient identifying means; and a doctor-side terminal device including a display device which displays the image of the patient selected by the image selecting means, and the image of the patient taken by the image taking device, such that the two images are comparable with each other by the doctor.

According to this aspect, the display device of the doctor-side terminal device displays the image selected from the respective images of the plurality of patients stored by the memory device, based on the set of patient identifying information inputted by the patient, and the image of the patient actually taken by the image taking device, such that the two images are comparable with each other. If the two images are recognized as being of the same person, the doctor can judge that the set of patient identifying information inputted by the patient is correct. Thus, the doctor can correctly identify the patient.

According to a second aspect of the present invention, there is provided a doctor-side terminal apparatus for use in a telediagnosis system for a doctor to make a diagnosis on a patient remote from the doctor, the apparatus comprising a display device which displays an image of the patient selected from respective pre-stored images of a plurality of patients based on a set of patient identifying information that identifies the patient, and an actually taken image of the patient, such that the two images are comparable with each other by the doctor.

Here, preferably, the display device of the doctor-side terminal device or apparatus displays the image of the patient selected based on the set of patient identifying information, and the actually taken image of the patient, such that the two images have a substantially same size and are arranged side by side. According to this feature, the two images can be easily compared, and accordingly the doctor can easily and correctly judge whether the two images are of the same patient.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a telediagnosis system to which the present invention is applied;
Fig. 2 is a schematic view showing the construction of a doctor's terminal device shown in Fig. 1;
Fig. 3 is a diagrammatic view for explaining essential control functions of a CPU (central processing unit) of the doctor's terminal device;
Fig. 4 is a view showing an example of an image displayed by a display device of the doctor's terminal device under control of a display control means shown in Fig. 3; and
Fig. 5 is a flow chart representing control functions related to a patient identifying means, an image selecting means, and the display control means that are shown in Fig. 3.

### DETAILED DESCRIPTION OF PREFERRED EMBODIMENTS

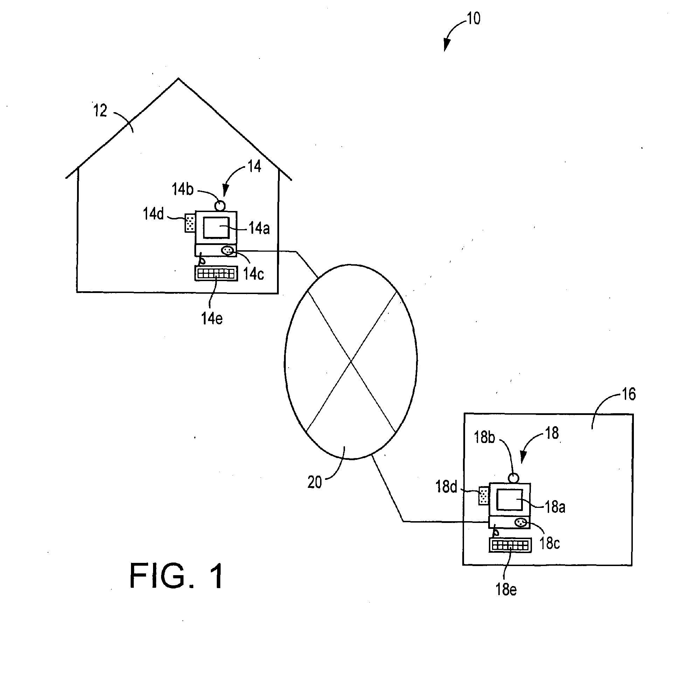

Hereinafter, there will be described a preferred embodiment of the present invention in detail by reference to the drawings. Fig. 1 is a view for explaining the construction of a telediagnosis system 10 to which the present invention is applied.

The telediagnosis system 10 includes a patient-side terminal device 14 that is disposed in an old-age home 12 as an example of a facility in which a plurality of patients live; a doctor-side terminal device 18 that is disposed in a medical-treatment facility 16 and is operated by a doctor; and a communication line 20, such as a telephone line, an internet, or a LAN (local area network), that can connect between the patient-side terminal device 14 and the doctor-side terminal device 18. Though Fig. 1 shows only one patient-side terminal device 14, the telediagnosis system 10 may employ a plurality of patient-side terminal devices 14. Likewise, the system 10 may employ a plurality of doctor-side terminal devices 18.

The patient-side terminal device 14 includes a display device 14a; a CCD (charge coupled device) camera 14b functioning as an image taking device; a microphone 14c; a speaker 14d; a keyboard 14e functioning as an input device; a memory device (not shown) such as a hard disk; a control device (not shown) that controls those elements 14a, 14b, 14c, 14d, 14e; and a communication device (not shown). Patients' face images taken by the CCD camera 14b and patients' voices and sounds inputted in the microphone 14c are sent via the communication line 20 to the doctor-side terminal device 18; and the doctor's face image and the doctor's voices and sounds received via the communication line 20 are outputted from the display device 14a and the speaker 14d, respectively.

The keyboard 14e can be operated for inputting a patient number that is given, in advance, to each patient and is used as patient information identifying the each patient.

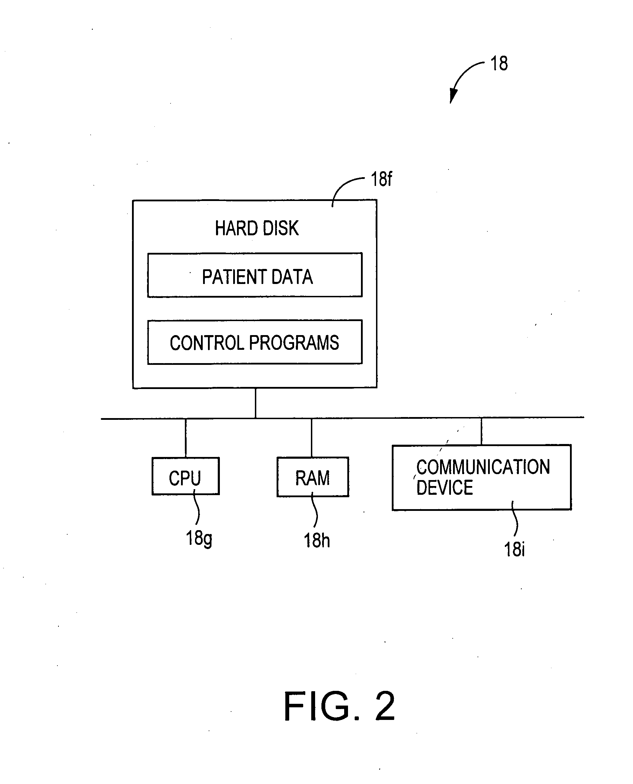

Like the patient-side terminal device 14, the doctor-side terminal device 18 includes a display device 18a; a CCD camera 18b; a microphone 18c; a speaker 18d; and a keyboard 18e, and additionally includes, as shown in Fig. 2 that schematically illustrates an internal construction of the device 14, a hard disk 18f, a CPU (central processing unit) 18g, a RAM (random access memory) 18h, and a communication device 18i.

The hard disk 18f functioning as a memory device stores a plurality of sets of patient data including a plurality of patient numbers, a plurality of sets of face-image data corresponding to the plurality of patient numbers, respectively, and a plurality of clinical-chart numbers corresponding to the plurality of patient numbers, respectively. The hard disk 18f additionally stores control programs that are used by the CPU 18g for controlling the display device 18a, the communication device 18i, etc. The CPU 18g operates for sending the doctor's face image taken by the CCD camera 18b and the doctor's voices and sounds inputted in the microphone 18c are sent via the communication line 20 to the patient-side terminal device 14; and the patients' face images and the patients', voices and sounds received via the communication line 20 are outputted from the display device 18a and the speaker 18d, respectively.

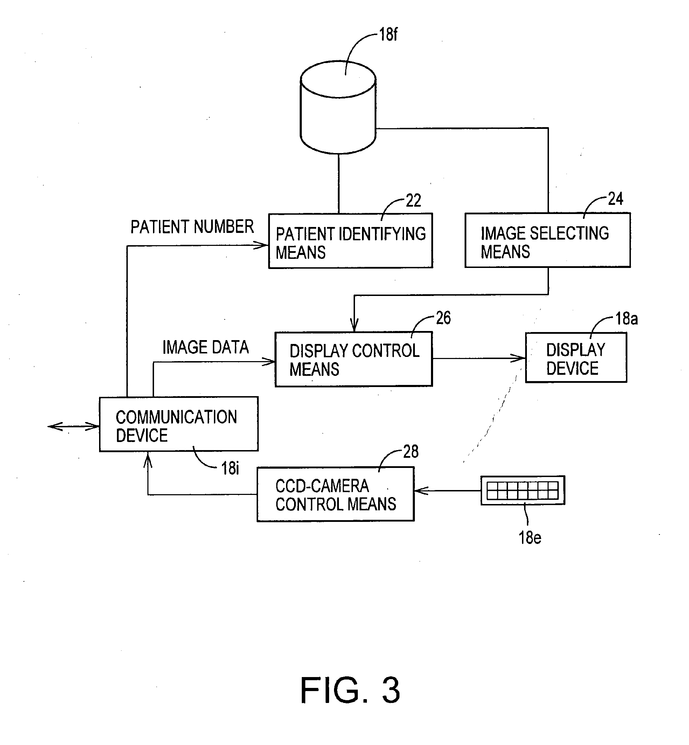

Fig. 3 is a diagrammatic view for explaining essential control functions of the CPU 18a of the doctor-side terminal device 18. A patient identifying device or means 22 checks the patient number sent from the patient-side terminal device 14, against the patient numbers as part of the patient data stored by the hard disk 18f, and thereby identifies a patient who has inputted the patient number (i.e., a clinical-chart number corresponding to the patient number).

An image selecting device or means 24 selects, from the plurality of sets of face-image data as another part of the patient data stored by the hard disk 18f, a set of face-image data representing the face of the patient identified by the patient identifying means 22. A display control device or means 26 operates the display device 18a to display the face image actually taken by the CCD camera 14b of the patient-side terminal device 14 (hereinafter, referred to as the "taken image"), based on the set of face-image data sent from the device 14, and the face image of the patient represented by the set of face-image data selected by the image selecting means 24 from the patient data stored by the hard disk 18f (hereinafter, referred to as the "stored image"), such that the two face images have a same size (i.e., a same scale factor) and are arranged side by side, so as to be comparable with each other by the doctor.

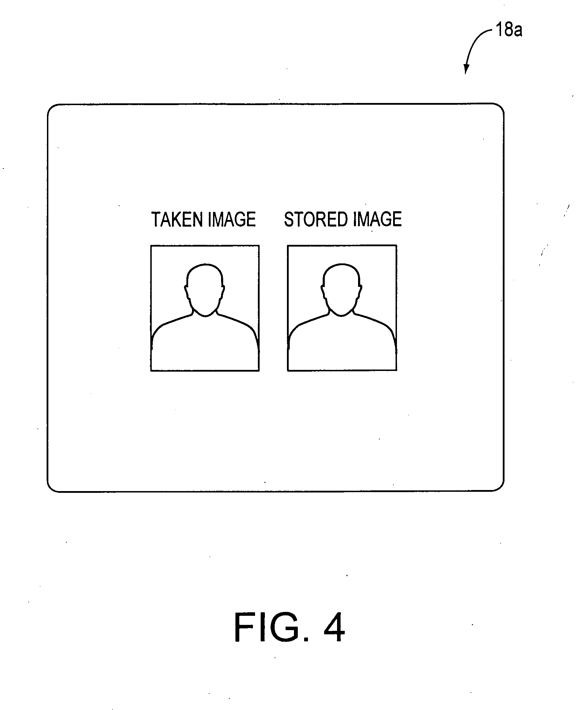

Fig. 4 shows an example of the two face images displayed by the display device 18a under control of the display control means 26. In the case where the display device 18a displays the taken image and the stored image in the same size, if the patient in front of the patient-side terminal device 14 is positioned in a corner of a field of view of the CCD camera 14b, or if the patient is too near to, or too far from, the camera 14b, the two faces represented by the two images do not have a same size.

To solve the above-indicated problem, a CCD-camera control device or means 28 enables the doctor to control the CCD camera 14b of the patient-side terminal device 14. More specifically described, the CCD-camera control means 28 sends, according to command signals inputted through the keyboard 18e by the doctor, control signals to control a field-of-view position, a scale factor, etc. of the CCD camera 14b of the patient-side terminal device 14.

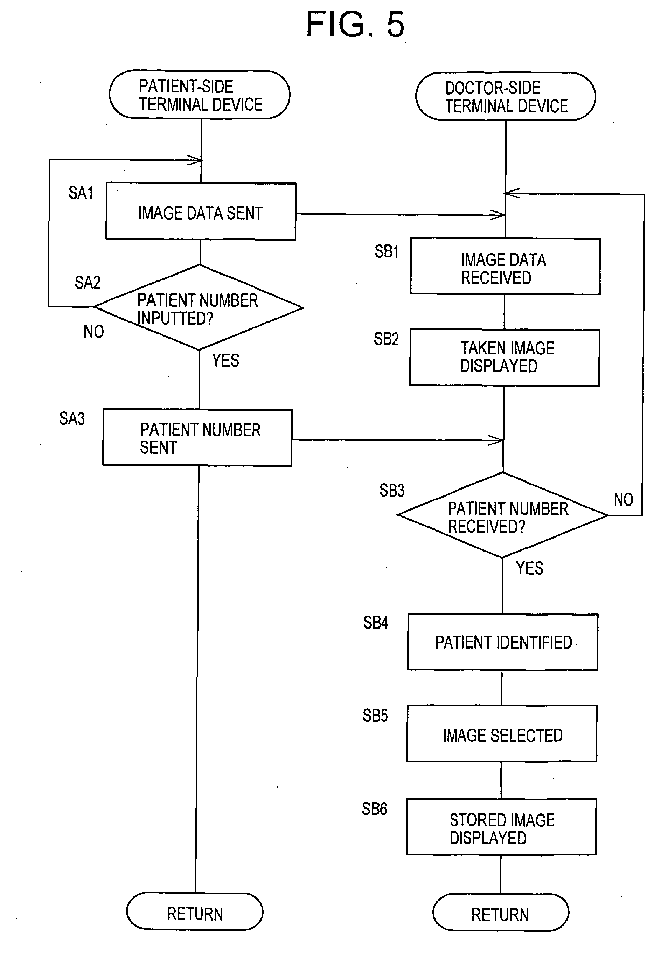

Fig. 5 is a flow chart representing the control operations of the CPU 18g, shown in Fig. 3, in particular, the patient identifying means 22, the image selecting means 24, and the display control means 26.

First, at Step SA1 (hereinafter, "Step" is omitted) on the patient-side terminal device 14, the set of face-image data representing the face of the patient taken by the CCD camera 14b, is sent to the doctor-side terminal device 18. Subsequently, at SA2, whether a patient number has been inputted through the keyboard 14e is judged. If a negative judgment is made at SA2, SA1 and the following step are repeated, while the set of face-image data is continuously sent to the doctor-side terminal device 18.

When the set of face-image data is sent to the doctor-side terminal device 18, the device 18 receives, at SB1, the set of face-image data and, at SB2, the CPU operates the display device 18a to display, based on the received set of face-image data, the taken image in a prescribed size, in a prescribed area of the device 18a. Subsequently, at SB3, the CPU judges whether the device 18 has received a patient number. If a negative judgment is made at SB3, SB1 and the following steps are repeated.

Meanwhile, if a positive judgment is made at SA2 on the patient-side terminal device 14, that is, if a patient number has been inputted, the control goes to SA3 to send the patient number inputted, to the doctor-side terminal device 18.

When the patient number is sent to the doctor-side terminal device 18, a positive judgment is made at SB3 on the device 18, and accordingly the control goes to SB4 where the CPU selects, from the plurality of patient numbers as part of the patient data stored in the hard disk 18f, the patient number identical with the patient number sent from the patient-side terminal device 14, and thereby identifies a patient (i.e., a clinical chart of the patient) corresponding to the patient number.

Subsequently, the control goes to SB5 corresponding to the image selecting means 24, where the CPU selects the set of face-image data corresponding to the patient number selected at SB4, and further goes to SB6 where the CPU operates the display device 18a to display, based on the set of face-image data selected at SB5, the stored image, such that the stored image and the taken image are arranged side by side and have a same size, as exemplarily shown in Fig. 4. Thus, in the flow chart shown in Fig. 4, SB1, SB2, and SB6 correspond to the display control means 26.

It emerges from the foregoing description that in the telediagnosis system 10, the display device 18a of the doctor-side terminal device 18 displays the face image of the patient actually taken by the CCD camera 14b, and the face image of the patient selected based on the patient number inputted by the patient from the plurality of face images stored in the hard disk 18f, such that the two images are arranged side by side and have a same size. If the doctor compares the two images and identifies that the two images represent a same person, the doctor can judge that the patient number inputted is correct, and thereby correctly identify the patient.

While the present invention has been described in its embodiment in detail by reference to the drawings, it is to be understood that the present invention can otherwise be embodied.

For example, in the illustrated telediagnosis system 10, the hard disk 18f as the memory device, the patient identifying means 22, and the image selecting means 24 are provided in the doctor-side terminal device 18. However, those elements 18f, 22, 24 may be provided in the patient-side terminal device 14. In the latter case, both the face image of the patient actually taken and the face image of the patient selected from the plurality of face images stored in the memory device are sent to the doctor-side terminal device 18 via the communication line 20.

In addition, the doctor-side terminal device 18 is provided with the CCD-camera control means 28. However, the doctor can instruct, via the device 18 and the patient-side terminal device 14, the patient to change the patient's position, so as to modify the size and position of the patient's face displayed on the display device 18a. In the latter case, the CCD-camera control means 28 may be omitted.

In addition, the display device 18a of the doctor-side terminal device 18 displays the taken image and the stored image such that the two images are arranged side by side, i.e., in left and right half areas, respectively, and have a same size. However, the two images may not have a same size, and may be displayed in top and bottom half areas, respectively, or in two oblique half areas, respectively.

In addition, in the illustrated telediagnosis system 10, the patient numbers are used as the sets of patient information. However, a fingerprint may be used as a set of patient information. Moreover, in the case where patient numbers are used, each patient number may not be inputted through the keyboard 14e, but may be recorded, in advance, on a recording medium, such as a card, so as to be read by a reading device.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A telediagnosis system (10) for a doctor to make a diagnosis on a patient remote from the doctor, the system comprising:
an image taking device (14b, 18e, 28, 18i, 20) which takes an image of said patient;
an input device (14e) which is operable for inputting a set of patient identifying information that identifies said patient;
a memory device (18f) which stores respective images of a plurality of patients, and a plurality of sets of patient identifying information that identify said plurality of patients, respectively;
a patient identifying means (22) for identifying said patient by checking the set of patient identifying information inputted through the input device, against the plurality of sets of patient identifying information stored by the memory device;
an image selecting means (24) for selecting, from the respective images of said plurality of patients stored by the memory device, the image of said patient identified by the patient identifying means; and
a doctor-side terminal device (18) including a display device (18a, 26) which displays the image of said patient selected by the image selecting means, and the image of said patient taken by the image taking device, such that the two images are comparable with each other by the doctor.

2. A doctor-side terminal apparatus (18) for use in a telediagnosis system (10) for a doctor to make a diagnosis on a patient remote from the doctor, the apparatus comprising:
a display device (18a, 26) which displays an image of said patient selected from respective pre-stored images of a plurality of patients based on a set of patient identifying information that identifies said patient, and an actually taken image of said patient, such that the two images are comparable with each other by the doctor.

3. A telediagnosis system according to claim 1, wherein the display device (18a, 26) displays the image of said patient selected by the image selecting means (24) and the image of said patient taken by the image taking device (14b, 18e, 28, 18i, 20), such that the two images have a substantially same size and are arranged side by side.

4. A doctor-side terminal apparatus according to claim 2, wherein the display device (18a, 26) displays the image of said patient selected based on the set of patient identifying information, and the actually taken image of said patient, such that the two images have a substantially same size and are arranged side by side.
